# EUROPEAN PATENT APPLICATION

(11) **EP 3 673 820 A1**
(43) Date of publication of application: **01.07.2020**
(21) Application number: 19218567.6
(22) Date of filing: 20.12.2019
(51) Int. Cl.: A61B 17/072

(54) **SURGICAL STAPLER BUTTRESS WITH TISSUE IN-GROWTH PROMOTION**

(30) Priority: 28.12.2018 US 201816235503
(71) Applicant: Ethicon LLC, Guaynabo 00969 (PR)
(72) Inventor: HEUPEL, Martin, 22851 Norderstedt (DE); MCGIVERON, Omar Z., Cincinnati, Ohio 45242 (US); VENDELY, Michael J., Cincinnati, Ohio 45242 (US); WONG, Jordan B., Cincinnati, Ohio 45242 (US)
(74) Representative: Carpmaels & Ransford LLP

(57) **Abstract**

A buttress assembly (322) for use with a surgical stapled site has a buttress and an adhesive on one side of the buttress. The buttress itself has a multi-layer construction with a mesh layer (326) on one side, a film layer (334) on the other side, and an adhesive film layer (332) in between the mesh layer and film layer. The mesh layer is a knit material with multiple openings that provide space for and promote tissue ingrowth with the buttress. As such the buttress is oriented in use so that the mesh layer faces and contact the tissue at the surgically stapled site. Opposite to the mesh layer, the film layer faces away from the tissue at the surgically stapled site and acts as an adhesion barrier.

## Description

### BACKGROUND

In some settings, endoscopic surgical instruments may be preferred over traditional open surgical devices since a smaller incision may reduce the post-operative recovery time and complications. Consequently, some endoscopic surgical instruments may be suitable for placement of a distal end effector at a desired surgical site through the cannula of a trocar. These distal end effectors may engage tissue in a number of ways to achieve a diagnostic or therapeutic effect (e.g., endocutter, grasper, cutter, stapler, clip applier, access device, drug/gene therapy delivery device, and energy delivery device using ultrasonic vibration, RF, laser, etc.). Endoscopic surgical instruments may include a shaft between the end effector and a handle portion, which is manipulated by the clinician. Such a shaft may enable insertion to a desired depth and rotation about the longitudinal axis of the shaft, thereby facilitating positioning of the end effector within the patient. Positioning of an end effector may be further facilitated through inclusion of one or more articulation joints or features, enabling the end effector to be selectively articulated or otherwise deflected relative to the longitudinal axis of the shaft.

Examples of endoscopic surgical instruments include surgical staplers. Some such staplers are operable to clamp down on layers of tissue, cut through the clamped layers of tissue, and drive staples through the layers of tissue to substantially seal the severed layers of tissue together near the severed ends of the tissue layers. Merely exemplary surgical staplers are disclosed in U.S. Pat. No. 4,805,823, entitled "Pocket Configuration for Internal Organ Staplers," issued February 21, 1989; U.S. Pat. No. 5,415,334, entitled "Surgical Stapler and Staple Cartridge," issued May 16, 1995; U.S. Pat. No. 5,465,895, entitled "Surgical Stapler Instrument," issued November 14, 1995; U.S. Pat. No. 5,597,107, entitled "Surgical Stapler Instrument," issued January 28, 1997; U.S. Pat. No. 5,632,432, entitled "Surgical Instrument," issued May 27, 1997; U.S. Pat. No. 5,673,840, entitled "Surgical Instrument," issued October 7, 1997; U.S. Pat. No. 5,704,534, entitled "Articulation Assembly for Surgical Instruments," issued January 6, 1998; U.S. Pat. No. 5,814,055, entitled "Surgical Clamping Mechanism," issued September 29, 1998; U.S. Pat. No. 6,978,921, entitled "Surgical Stapling Instrument Incorporating an E-Beam Firing Mechanism," issued December 27, 2005; U.S. Pat. No. 7,000,818, entitled "Surgical Stapling Instrument Having Separate Distinct Closing and Firing Systems," issued February 21, 2006; U.S. Pat. No. 7,143,923, entitled "Surgical Stapling Instrument Having a Firing Lockout for an Unclosed Anvil," issued December 5, 2006; U.S. Pat. No. 7,303,108, entitled "Surgical Stapling Instrument Incorporating a Multi-Stroke Firing Mechanism with a Flexible Rack," issued December 4, 2007; U.S. Pat. No. 7,367,485, entitled "Surgical Stapling Instrument Incorporating a Multistroke Firing Mechanism Having a Rotary Transmission," issued May 6, 2008; U.S. Pat. No. 7,380,695, entitled "Surgical Stapling Instrument Having a Single Lockout Mechanism for Prevention of Firing," issued June 3, 2008; U.S. Pat. No. 7,380,696, entitled "Articulating Surgical Stapling Instrument Incorporating a Two-Piece E-Beam Firing Mechanism," issued June 3, 2008; U.S. Pat. No. 7,404,508, entitled "Surgical Stapling and Cutting Device," issued July 29, 2008; U.S. Pat. No. 7,434,715, entitled "Surgical Stapling Instrument Having Multistroke Firing with Opening Lockout," issued October 14, 2008; U.S. Pat. No. 7,721,930, entitled "Disposable Cartridge with Adhesive for Use with a Stapling Device," issued May 25, 2010; U.S. Pat. No. 8,408,439, entitled "Surgical Stapling Instrument with An Articulatable End Effector," issued April 2, 2013; and U.S. Pat. No. 8,453,914, entitled "Motor-Driven Surgical Cutting Instrument with Electric Actuator Directional Control Assembly," issued June 4, 2013. The disclosure of each of the above-cited U.S. Patents is incorporated by reference herein.

While the surgical staplers referred to above are described as being used in endoscopic procedures, it should be understood that such surgical staplers may also be used in open procedures and/or other non-endoscopic procedures. By way of example only, a surgical stapler may be inserted through a thoracotomy, and thereby between a patient's ribs, to reach one or more organs in a thoracic surgical procedure that does not use a trocar as a conduit for the stapler. Such procedures may include the use of the stapler to sever and close a vessel leading to a lung. For instance, the vessels leading to an organ may be severed and closed by a stapler before removal of the organ from the thoracic cavity. Of course, surgical staplers may be used in various other settings and procedures.

Examples of surgical staplers that may be particularly suited for use through a thoracotomy are disclosed in U.S. Pat. No. 9,186,142, entitled "Surgical Instrument End Effector Articulation Drive with Pinion and Opposing Racks," issued November 17, 2015; U.S. Pat. No. 9,717,497, entitled "Lockout Feature for Movable Cutting Member of Surgical Instrument," issued August 1, 2017; U.S. Pat. No. 9,517,065, entitled "Integrated Tissue Positioning and Jaw Alignment Features for Surgical Stapler," issued December 13, 2016; U.S. Pat. No. 9,839,421, entitled "Jaw Closure Feature for End Effector of Surgical Instrument," issued December 12, 2017; U.S. Pat. No. 9,867,615, entitled "Surgical Instrument with Articulation Lock having a Detenting Binary Spring," issued January 16, 2018; U.S. Pat. No. 9,622,746, entitled "Distal Tip Features for End Effector of Surgical Instrument," issued April 18, 2017; U.S. Pat. No. 10,092,292, entitled "Staple Forming Features for Surgical Stapling Instrument," issued October 9, 2018; U.S. Pat. No. 9,795,379, entitled "Surgical Instrument with Multi-Diameter Shaft," issued October 24, 2017; and U.S. Pat. No. 9,808,248, entitled "Installation Features for Surgical Instrument End Effector Cartridge," issued November 7, 2017. The disclosure of each of the above-cited U.S. Patent Publications is incorporated by reference herein.

Additional surgical stapling instruments are disclosed in U.S. Pat. No. 8,801,735, entitled "Surgical Circular Stapler with Tissue Retention Arrangements," issued August 12, 2014; U.S. Pat. No. 8,141,762, entitled "Surgical Stapler Comprising a Staple Pocket," issued March 27, 2012; U.S. Pat. No. 8,371,491, entitled "Surgical End Effector Having Buttress Retention Features," issued February 12, 2013; U.S. Pat. No. 9,597,082, entitled "Method and Apparatus for Sealing End-to-End Anastomosis" issued March 21, 2017; U.S. Pat. No. 9,398,911, entitled "Rotary Powered Surgical Instruments with Multiple Degrees of Freedom," issued July 26, 2016; U.S. Pat. Pub. No. 2013/0206813, entitled "Linear Stapler," published August 15, 2013; U.S. Pat. Pub. No. 2008/0169328, entitled "Buttress Material for Use with a Surgical Stapler," published July 17, 2008; U.S. Pat. No. 9,848,871, entitled "Woven and Fibrous Materials for Reinforcing a Staple Line," issued December 26, 2017; U.S. Pat. No. 9,936,954, entitled "Devices and Methods for Sealing Staples in Tissue" issued April 10, 2018; and U.S. Pat. Pub. No. 2016/0089146, entitled "Radically Expandable Staple Line" published March 31, 2016. The disclosure of each of the above-cited U.S. Patents, U.S. Patent Publications, and U.S. Patent Applications is incorporated by reference herein.

In some instances, it may be desirable to equip a surgical stapling instrument with a buttress material to reinforce the mechanical fastening of tissue provided by staples. Such a buttress may prevent the applied staples from pulling through tissue and may otherwise reduce a risk of tissue tearing at or near the site of applied staples.

While various kinds of surgical stapling instruments and associated components have been made and used, it is believed that no one prior to the inventor(s) has made or used the invention described in the appended claims.

Buttress assemblies for reinforcing tissue layers joined by surgical stapling are provided. The mesh layer may have a plurality of openings that are configured to promote tissue ingrowth. The plurality of openings may be configured in size and/or number such that they promote tissue ingrowth when one side of mesh layer of buttress is placed adjacent or in contact with tissue at a stapled site. Such tissue ingrowth can provide strength to the cut and stapled site and promote healing of the tissue.

The first film layer may be configured as an adhesive layer. The first film layer may be located on one side of mesh layer, and may be configured to adhesively attach mesh layer with the second film layer.

The second film layer may be configured as an adhesion barrier to prevent tissue adhesions from forming at or near the surgically stapled site. The second film layer may be configured to receive adhesive. The second film layer is on the side of buttress that releasably attaches with end effector when buttress assemblies are loaded to end effector.

### BRIEF DESCRIPTION OF THE DRAWINGS

The accompanying drawings, which are incorporated in and constitute a part of this specification, illustrate embodiments of the invention, and, together with the general description of the invention given above, and the detailed description of the embodiments given below, serve to explain the principles of the present invention.
FIG. 1 depicts a perspective view of an exemplary end effector of a surgical stapler and an exemplary buttress applicator, with the end effector approaching the buttress applicator;
FIG. 2 depicts a perspective view of the end effector and the buttress applicator of FIG. 1, with the buttress applicator positioned in the end effector;
FIG. 3A depicts a cross-sectional end view of a portion of the end effector of FIG. 1 with the buttress assembly of FIG. 1 applied to the end effector, with tissue positioned between the buttresses in the end effector, and with the anvil in an open position;
FIG. 3B depicts a cross-sectional end view of the combined end effector and buttress assembly of FIG. 3A, with tissue positioned between the buttresses in the end effector, and with the anvil in a closed position;
FIG. 3C depicts a cross-sectional view of a staple and the buttress assembly of FIG. 3A having been secured to the tissue by the end effector of FIG. 1;
FIG. 4 depicts a perspective view of staples and the buttress assembly of FIG. 3A having been secured to the tissue by the end effector of FIG. 1;
FIG. 5 depicts a perspective view of another exemplary buttress applicator usable with the end effector of FIG. 1;
FIG. 6 depicts an exploded view of the buttress applicator of FIG. 5;
FIG. 7 depicts a perspective view of an exemplary buttress of the buttress assembly of the buttress applicator of FIG. 5, showing a mesh layer.
FIG. 8 depicts an enlarged view of the mesh layer of the buttress of FIG. 7;
FIG. 9 depicts a perspective view of an exemplary buttress assembly of the buttress applicator of FIG. 5, showing a film layer with adhesive thereon; and
FIG. 10 depicts a cross section view of the buttress of FIG. 7 taken along line 10-10 as shown in FIG. 7.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

### I. Exemplary Buttress Loading and Application

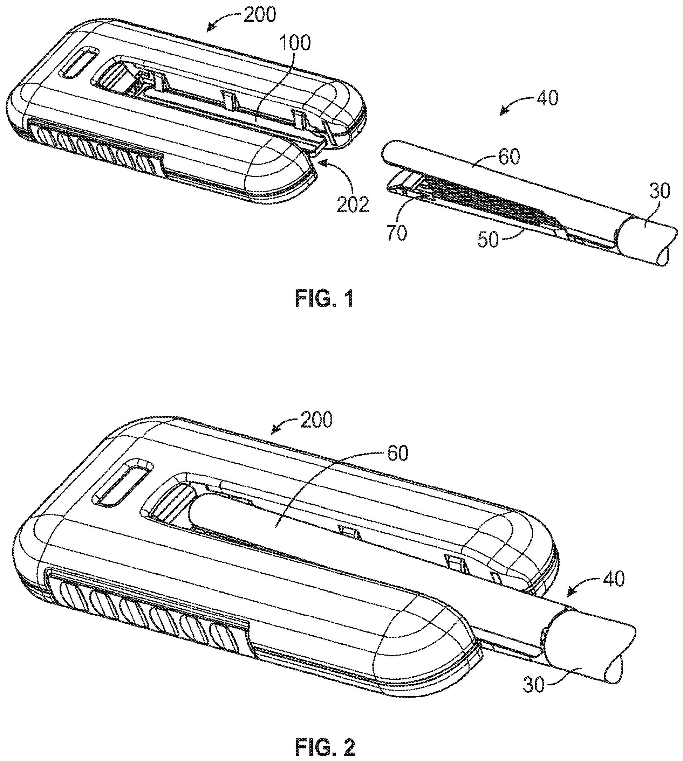

FIGS. 1 and 2 illustrate an exemplary end effector (40) configured to apply a buttress to a tissue site where a cutting and stapling operation is performed. End effector (40) is connected with a shaft assembly (30). End effector (40) comprises an anvil (60), a lower jaw (50), and a staple cartridge (70) received by lower jaw (50).

FIGS. 1 and 2 also illustrate an exemplary buttress applicator (200). Buttress applicator (200) is configured to selectively retain buttress assemblies (100, 110). In the present example, buttress assembly (100) is selectively retained on a top side of applicator (200) and buttress assembly (110) is selectively retained on a bottom side of applicator (200). In some other versions, applicator (200) can be configured such that only one buttress assembly (100, 110) is selectively retained by buttress applicator (200).

To use buttress applicator (200) to load end effector (40) with buttress assemblies (100, 110), the operator would first position applicator (200) and end effector (40) such that end effector (40) is aligned with an open end (202) of applicator (200) as shown in FIG. 1. The operator would then advance end effector (40) distally (and/or retract applicator (200) proximally) to position buttress assemblies (100, 110) between anvil (60) and staple cartridge (70) as shown in FIG. 2.

In order to load buttress assemblies (100, 110) on end effector (40), the operator may simply close end effector (40) by pivoting anvil (60) toward staple cartridge (70). Closure of end effector (40) results in the distal ends of anvil (60) and staple cartridge (70) bearing against retaining features of buttress applicator (200) that are configured to selectively retain buttress assemblies (100, 110) with buttress applicator (200). This contact deflects such retaining features of buttress applicator (200) to thereby permit contact between a surface of anvil (60) and buttress assembly (100) on one side of buttress applicator (200), and a surface of staple cartridge (70) and buttress assembly (110) on another side of buttress applicator (200).

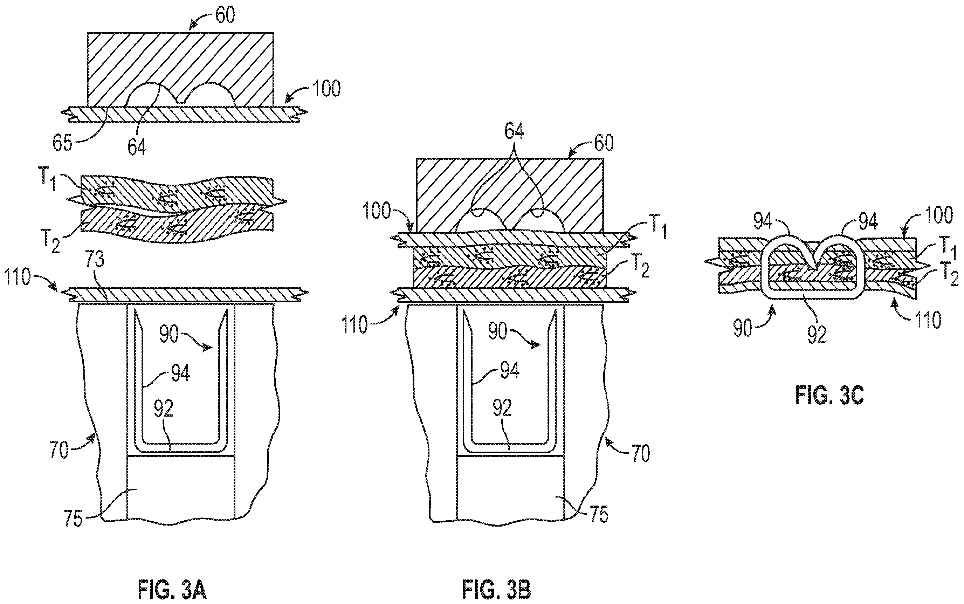

Buttress assemblies (100, 110) comprise an adhesive on their respective surfaces such that with end effector (40) clamping on both buttress assemblies (100, 110), buttress assemblies (100, 110) are adhered respectively to an underside of anvil (60) and a deck surface of staple cartridge (70). End effector (40) may then be re-opened (i.e., pivoting anvil (60) away from staple cartridge (70)) and pulled away from buttress applicator (200). With retaining features of applicator (200) disengaged from buttress assemblies (100, 110), end effector (40) may freely pull buttress assemblies (100, 110) away from buttress applicator (200) as end effector (40) is pulled away from buttress applicator (200). With buttress assemblies (100, 110) loaded on end effector (40), end effector (40) may then be used as described further below with reference to FIGS. 3A-4.

FIGS. 3A-3C show a sequence where an end effector (40) that has been loaded with buttress assemblies (100, 110) is actuated to drive staples (90) through two apposed layers of tissue (T₁, T₂), with buttress assemblies (100, 110) being secured to the same layers of tissue (T₁, T₂) by staples (90). In particular, FIG. 3A shows layers of tissue (T₁, T₂) positioned between anvil (60) and staple cartridge (70), with anvil (60) in the open position. As shown, anvil (60) comprises staple forming pockets (64). Buttress assembly (100) is adhered, via adhesive, to underside (65) of anvil (60); while buttress assembly (110) is adhered, via adhesive, to deck (73) of staple cartridge (70). Layers of tissue (T₁, T₂) are thus interposed between buttress assemblies (100, 110). Next, end effector (40) is closed, which drives anvil (60) to the closed position as shown in FIG. 3B. At this stage, layers of tissue (T₁, T₂) are compressed between anvil (60) and staple cartridge (70), with buttress assemblies (100, 110) engaging opposite surfaces of tissue layers (T₁, T₂). End effector (40) is then actuated, whereby a staple driver (75) drives staple (90) through buttress assemblies (100, 110) and tissue layers (T₁, T₂). As shown in FIG. 3C, crown (92) of driven staple (90) captures and retains buttress assembly (110) against layer of tissue (T₂). Deformed legs (94) of staple (90) capture and retain buttress assembly (100) against layer of tissue (T₁).

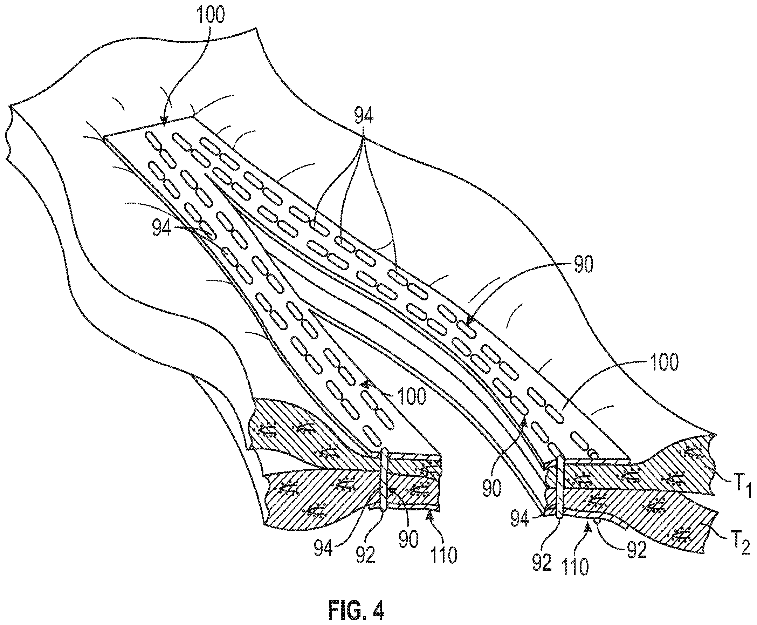

It should be understood that a series of staples (90) will similarly capture and retain buttress assemblies (100, 110) against layers of tissue (T₁, T₂), thereby securing buttress assemblies (100, 110) to tissue (T₁, T₂) as shown in FIG. 4. As end effector (40) is pulled away from tissue (90) after deploying staples (90) and buttress assemblies (100, 110), buttress assemblies (100, 110) disengage end effector, such that buttress assemblies (100, 110) remain secured to tissue (T₁, T₂) with staples (90). Buttress assemblies (100, 110) thus provide structural reinforcement to the lines of staples (90). As can also be seen in FIG. 4, a knife member (not shown) passes through end effector (40) and in doing so also cuts through a centerline of buttress assemblies (100, 110), separating each buttress assembly (100, 110) into a corresponding pair of sections, such that each section remains secured to a respective severed region of tissue (T₁, T₂).

In the foregoing example, buttress assembly (100) is sized to span across the full width of underside (65) of anvil (60), such that a knife member (not shown) cuts through buttress assembly (100) during actuation of end effector (40). In some other examples, buttress assembly (100) is provided in two separate, laterally spaced apart portions, with one portion being disposed on underside (65) of anvil (60) on one half of anvil (60) and another portion being disposed on underside (65) of anvil (60) on the other half of anvil (60). In such versions, the knife member (not shown) does not cut through buttress assembly (100) during actuation of end effector (40).

Likewise, buttress assembly (110) may be sized to span across the full width of deck (73), such that the knife member (not shown) cuts through buttress assembly (110) during actuation of end effector (40). Alternatively, buttress assembly (110) may be provided in two separate, laterally spaced apart portions, with one portion being disposed on deck (73) on one half and another portion being disposed on deck (73) on the other half. In such versions, the knife member (not shown) does not cut through buttress assembly (110) during actuation of end effector (40).

In addition to the foregoing, it should also be understood that any of the various buttress assemblies described herein may be further constructed and operable in accordance with at least some of the teachings of U.S. Pat. Pub. No. 2016/0278774, entitled "Method of Applying a Buttress to a Surgical Stapler," published September 29, 2016, the disclosure of which is incorporated by reference herein.

### II. Exemplary Buttress Applicator

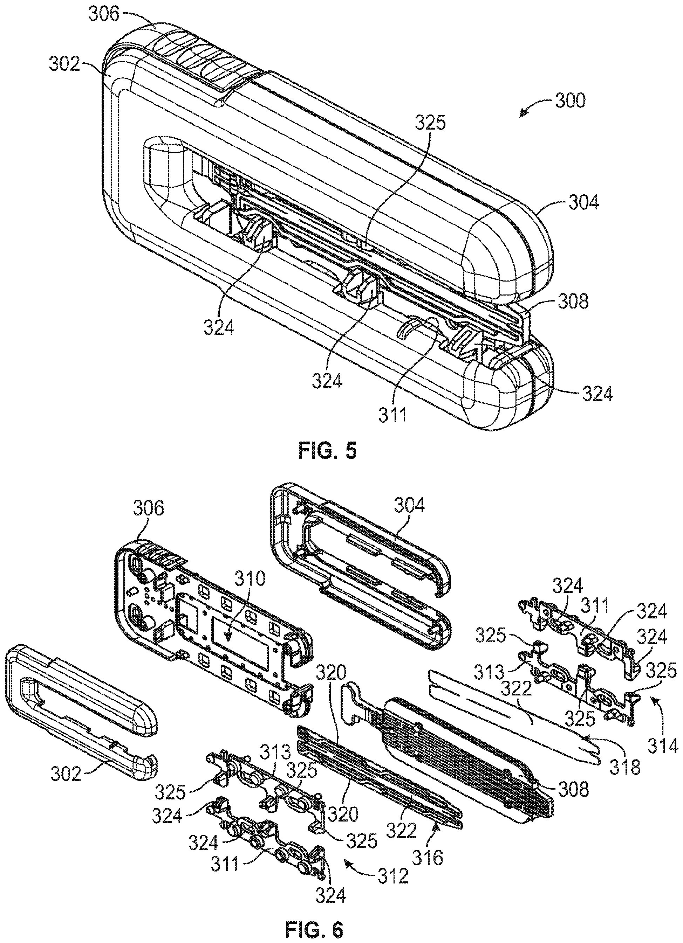

FIGS. 5 and 6 illustrate an alternate buttress applicator (300) for use with end effector (40). Buttress applicator (300) comprises a first housing portion (302) and a second housing portion (304). Each of housing portions (302, 304) connects with a frame (306). A compression pad (308) is configured to fit within a central portion (310) of frame (306). A first pair of clamp arms (312) are located on a first side of frame (306) between frame (306) and housing portion (302). A second pair of clamp arms (314) are located on a second side of frame (306) between frame (306) and housing portion (304). In the present version, clamp arms (312) comprise a left clamp arm (311) and a right clamp arm (313). Similarly, clamp arms (314) comprise a left clamp arm (311) and a right clamp arm (313). Buttress assemblies (316, 318) are located on respective sides of compression pad (308), and when buttress applicator (300) is fully assembled, pairs of clamp arms (312, 314) selectively retain buttress assemblies (316, 318) against compression pad (308). In the present example buttress assemblies (316, 318) are the same with each comprising an adhesive (320) located on a buttress (322) as will be described in greater detail below.

Buttress applicator (300) can be used with end effector (40) in the same manner as described above with respect to buttress applicator (200). For instance, buttress assemblies (316, 318) are loaded to end effector (40) in the same manner as described above where end effector (40) is moved to a closed or clamped position once anvil (60) and lower jaw (50) are positioned over central portion (310) of frame (308), e.g. as illustrated in FIG. 2. More specifically, the clamping action of end effector (40) when over buttress assemblies (316, 318) and compression pad (308) causes anvil (60) and staple cartridge (70) of lower jaw (50) to contact retention features (324) on left clamp arms (311) and retention features (325) on right clamp arms (313). This contact drives clamp arms (311, 313) laterally away from buttress assemblies (316, 318) thereby disengaging retention features (324, 325) from buttress assemblies (316, 318). With retention features (324, 325) disengaged, depending on the clamping orientation used with end effector (40), adhesive (320) of buttress assembly (316) contacts either underside (65) of anvil (60) or deck (73) of staple cartridge (70), while adhesive (320) of buttress assembly (318) contacts the other of underside (65) of anvil (60) or deck (73) of staple cartridge (70). This causes buttress assemblies (316, 318) to attach with end effector (40) and remain with end effector (40) as end effector is opened and moved away from buttress applicator (300). From this point, buttress assemblies (316, 318) may be applied to a cut and staple tissue site as described above and illustrated with respect to FIG. 4.

### III. Exemplary Buttress Construction and Arrangement

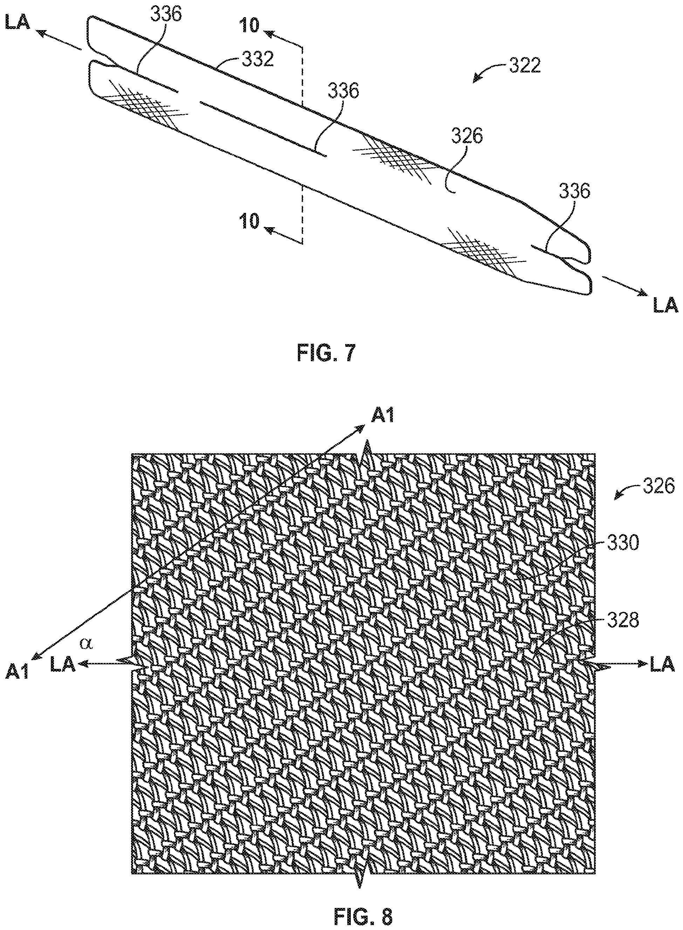

FIGS. 7 and 8 illustrate buttress (322) of buttress assemblies (316, 318). Buttress (322) comprises a multi-layer material that is laminated together. In the present example, buttress (322) comprises a mesh layer (326) and two film layers (332, 334). Mesh layer (326) comprises a knitted synthetic absorbable material. In some examples, mesh layer (326) is knit using yarns (328) comprised of polyglactin 910, which is 90% glycolide and 10% L-lactide. An example of polyglactin 910 is manufactured by Ethicon Inc. under the brand name Vicryl®. In view of the teachings herein, other absorbable synthetic materials for use with mesh layer (326) will be apparent to those of ordinary skill in the art.

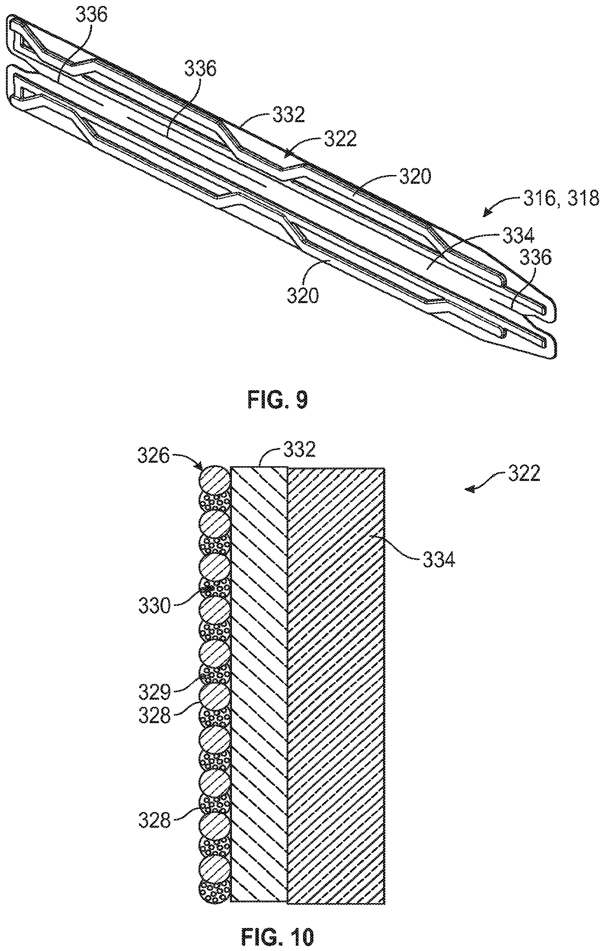

FIG. 8 illustrates an enlarged portion of mesh layer (326), showing an exemplary knit pattern produced by the arrangement of yarns (328). In the present example, each yarn (328) comprises a bundle or structure having 28 individual filaments. However, in other versions each yarn (328) can comprise a single filament or a multifilament bundle with greater or fewer than 28 individual filaments. An example of the multifilament structures of mesh layer (326) can be seen in FIG. 10, where the uncut yarns in cross section illustrate multiple filaments (329) that make up the multifilament structure of yarns (328).

As best seen in FIG. 8, mesh layer (326) comprises a plurality of openings (330) defined by the knit yarns (328). With its openings (330) mesh layer (326) comprises a porosity. Depending on the number and/or size of openings (330) the porosity of mesh layer (326) can be changed with various mesh layers (326). Furthermore, the knit pattern as well as the yarn size can influence openings (330) and the resultant porosity. In the present example, plurality of openings (330) are configured in size and/or number such that they promote tissue ingrowth when one side of mesh layer (326) of buttress (322) is placed adjacent or in contact with tissue at a stapled site. In this manner, buttress (322) comprises one or more tissue ingrowth features, which are configured to promote tissue ingrowth with buttress (322). Such tissue ingrowth can provide strength to the cut and stapled site and promote healing of the tissue. In view of the teachings herein, various ways to modify mesh layer (328) to achieve a desired porosity, and/or size of openings (330), and/or number of openings (330), will be apparent to those of ordinary skill in the art in view of the teachings herein.

Referring now to FIG. 10, as mentioned above, buttress (322) also comprises a film layer (332). Film layer (332) is configured as an adhesive layer. In the present example, film layer (332) is located on one side of mesh layer (326), and is configured to adhesively attach mesh layer (326) with film layer (334). Film layer (332), in the present example, comprises a polydioxanone (PDO) film; however, in other versions other types of film may be used and will be apparent to those of ordinary skill in the art in view of the teachings herein. Furthermore, film layer (332) is about 8 micrometers in thickness in the present example; however, in other versions the thickness of film layer (332) may be greater or less than 8 micrometers. In some versions, the polydioxanone film can be included in the construct as a thin film either on its own at the time of lamination or coextruded onto film layer (334) prior to lamination to the mesh layer (326).

Referring still to FIG. 10, and also FIG. 9, film layer (332) is also in contact with film layer (334). Film layer (334) contacts film layer (332) on the side opposite to where film layer (332) contacts mesh layer (326). In the present example, film layer (334) is configured as an adhesion barrier to prevent tissue adhesions from forming at or near the surgically stapled site. Film layer (334), in the present example, is constructed of poliglecaprone 25. An example poliglecaprone 25 is manufactured by Ethicon Inc. under the brand name Moncryl®. In other versions, other types of film may be used and will be apparent to those of ordinary skill in the art in view of the teachings herein. In the present example, film layer (334) is about 10 micrometers in thickness; however, in other versions the thickness of film layer (334) may be greater or less than 10 micrometers.

Film layer (334) is also substantially continuous in the present example, and is configured to receive adhesive (320) as described above and as shown best in FIG. 9. With this configuration, film layer (334) is on the side of buttress (322) that releasably attaches with end effector (40) when buttress assemblies (316, 318) are loaded to end effector (40) as described above. Furthermore, with this configuration, after actuating end effector (40) in a stapling action, film layer (334) is positioned facing away from the stapled tissue site, whereas openings (330) of mesh layer (326) are positioned facing the stapled tissue site as mentioned above to promote tissue ingrowth. While film layer (334) is illustrated as substantially continuous, with the exception of slits (336) described further below, in some other versions film layer (334) can include openings therein, for example as disclosed in U.S. Pat. No. 8,579,990, entitled "Tissue Repair Devices of Rapid Therapeutic Absorbency," issued November 12, 2013, and incorporated by reference herein.

When constructing buttress (322), mesh layer (326) and film layers (332, 334) are laminated together to form the multi-layer material comprising buttress (322). In one version, the lamination occurs under elevated pressure and temperature. Buttress (322) is subjected to these conditions for a predetermined time to achieve acceptable lamination of the layers.

In the present example, buttress (322) is mechanically cut from a larger piece or fabric of multi-layer material having the same construction as described above for buttress (322). This mechanical cutting can be done at lower temperatures compared to other cutting applications, which allows for buttresses (322) to be cut from larger fabrics while avoiding charred or burnt edges that can occur with heated cutting techniques.

Also in the present example, buttresses (322) are mechanically cut out from the larger fabric such that the knit pattern is cut along a 45 degree angle relative to a longitudinal axis (LA) of buttress (322). Referring to FIGS. 7 and 8, the knit pattern of mesh layer (326) is illustrated having been cut out at about 45 degrees relative to longitudinal axis (LA). For instance, axis (A1) illustrates the direction of the knit pattern of mesh layer (326). Furthermore, the intersection of axis (A1) and longitudinal axis (LA) of buttress (322) forms an angle (α), which is approximately 45 degrees in the present example. By using this configuration for the cut out angle, unraveling of the mesh layer (326) of cut out buttress (322) can be avoided. In view of the teachings herein, other cut out angles and ways to prevent unraveling of a cut mesh layer (326) will be apparent to those of ordinary skill in the art.

Referring again to FIGS. 7 and 9, buttress (322) also comprises multiple slits (336) that extend longitudinally along buttress (322). Slits (336) extend all the way through mesh layer (326), through film layer (332), and through film layer (334). Furthermore, slits (336) are positioned to generally divide buttress (332) into two equal sections. Referring to FIG. 4, when buttress assemblies (316, 318) are used with end effector (40) in a cutting and stapling procedure, the knife (not shown) of the stapler will travel longitudinally down end effector (40) to cut clamped tissue and at the same time cut buttresses (322) of buttress assemblies (316, 318) along slits (336). This creates the cut and stapled site as illustrated in FIG. 4. Slits (336) act as precuts in buttress (332) such that during a cutting and stapling action, buttress (332) offers less resistance to being cut, which promotes buttress (332) remaining properly placed relative to the surgically cut and stapled site, instead of buttress (332) being pushed longitudinally by the cutting knife and bunching. While the present example uses slits (336) to precut and promote ease of cutting buttress (322), in view of the teachings herein, other techniques and precut geometries can be used and will be apparent to those of ordinary skill in the art.

### IV. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A buttress assembly, for reinforcing tissue layers joined by surgical stapling, comprises (a) a multi-layer base material comprising (i) a mesh layer comprising a first mesh surface and a second mesh surface, wherein the first mesh surface is configured to contact the tissue joined by the surgical stapling, (ii) a first film layer contacting the second mesh surface of the mesh layer, wherein the first film layer is configured as an adhesive layer, and (iii) a second film layer comprising a first film surface and a second film surface, wherein first film surface contacts the first film layer, wherein the second film surface is configured to face away from the tissue joined by the surgical stapling; and (b) an adhesive located on the second film surface of the second film layer, wherein the adhesive is configured to provide releasable adhesion of the buttress assembly with an end effector of a surgical stapler.

### Example 2

The buttress assembly of Example 1, wherein the mesh layer comprises a knitted textile constructed from an absorbable synthetic material.

### Example 3

The buttress assembly of any one or more of Examples 1 and 2, wherein the mesh layer comprises polyglactin 910.

### Example 4

The buttress assembly of any one or more of Examples 1 through 3, wherein the mesh layer comprises a multi-filament structure.

### Example 5

The buttress assembly of any one or more of Examples 1 through 4, wherein the first film layer comprises polydioxanone.

### Example 6

The buttress assembly of any one or more of Examples 1 through 5, wherein the first film layer has a thickness of about eight micrometers.

### Example 7

The buttress assembly of any one or more of Examples 1 through 6, wherein the second film layer comprises an absorbable synthetic material.

### Example 8

The buttress assembly of any one or more of Examples 1 through 7, wherein the second film layer comprises poliglecaprone 25.

### Example 9

The buttress assembly of any one or more of Examples 1 through 8, wherein the second film layer has a thickness of about ten micrometers.

### Example 10

The buttress assembly of any one or more of Examples 1 through 9, wherein the second film layer is configured as an adhesion barrier to prevent tissue adhesions.

### Example 11

The buttress assembly of any one or more of Examples 1 through 10, wherein the multi-layer base material is formed by laminating the mesh layer, the first film layer, and the second film layer together by applying a predetermined pressure and predetermined heat over a predetermined time.

### Example 12

The buttress assembly of any one or more of Examples 1 through 11, wherein the multi-layer base material of the buttress assembly comprises cut edges.

### Example 13

The buttress assembly of any one or more of Examples 1 through 12, wherein the mesh layer defines a knit pattern, wherein the knit pattern is oriented at about 45 degrees relative to a longitudinal axis of the buttress assembly.

### Example 14

The buttress assembly of any one or more of Examples 1 through 13, wherein the mesh layer comprises a plurality of openings that are configured to promote tissue ingrowth.

### Example 15

The buttress assembly of any one or more of Examples 1 through 14, wherein the plurality of openings are defined by a knitted textile constructed from an absorbable synthetic material.

### Example 16

A buttress, configured for use with an end effector of a surgical stapler, is further configured for reinforcing a surgically stapled site. The buttress comprises (a) a mesh layer knitted from a multi-filament absorbable synthetic material, wherein the mesh layer defines a plurality of openings. On a first side of the mesh layer the plurality of openings are configured to face the surgically stapled site and contact tissue to promote tissue ingrowth. The buttress further comprises (b) a film layer located opposite to the first side of the mesh layer with the exposed plurality of openings, wherein the film layer is configured to face away from the surgically stapled site and prevent tissue adhesions at and around the surgically stapled site.

### Example 17

The buttress of Example 16, wherein the mesh layer comprises polyglactin 910, and wherein the film layer comprises poliglecaprone 25.

### Example 18

The buttress of any one or more of Examples 16 and 17, further comprising an adhesive layer positioned between the mesh layer and the film layer, wherein the adhesive layer is configured to adhesively bond the mesh layer with the film layer to form a laminate, wherein the adhesive layer comprises polydioxanone.

### Example 19

A method of reinforcing tissue at a surgically stapled site comprises (a) positioning an end effector of a surgical stapler relative to a buttress applicator. The buttress applicator selectively retains a buttress comprising (i) a mesh layer knitted from a multi-filament absorbable synthetic material. The mesh layer defines a plurality of openings, wherein on a first side of the mesh layer the plurality of openings are configured to face the surgically stapled site and contact tissue to promote tissue ingrowth. The buttress further comprises (ii) a film layer located opposite to the first side of the mesh layer with the exposed plurality of openings. The film layer is configured to face away from the surgically stapled site and prevent tissue adhesions at and around the surgically stapled site. The method further comprises (b) clamping the end effector onto a portion of the buttress applicator, wherein the act of clamping causing disengagement of retention features of the buttress applicator from the buttress. The act of clamping further causes the buttress to releasably attach with the end effector via an adhesive located on the buttress. The method further comprises (c) separating the end effector from the buttress applicator, (d) positioning the end effector relative to the tissue to be surgically stapled, and (e) stapling the tissue. The act of stapling the tissue comprises applying the buttress to the surgically stapled site with the first side of the mesh layer facing the surgically stapled site, and with the film layer facing away from the surgically stapled site to act as an adhesion barrier.

### Example 20

The method of Example 19, further comprising prior to the buttress being loaded onto the buttress applicator, the buttress is mechanical cut from a larger piece, wherein the cutting avoids overheating the buttress, thereby preventing charred edges

### V. Miscellaneous

While the terms "buttress" and "buttress assembly" are used throughout this disclosure, it should be understood that the term is not intended to limit the scope of the present invention in any way. For instance, use of the terms "buttress" and "buttress assembly" is not intended to demonstrate contemplation that a "buttress" or "buttress assembly" can only be used to provide structural support to a staple line or serve any other particular purpose. It is contemplated that "buttress" or "buttress assembly" may serve a variety of purposes in addition to or as an alternative to providing structural support to a staple line. The terms "buttress" and "buttress assembly" should therefore be read broadly to include any kind of adjunct to a staple line that serves any suitable purpose.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

In addition to the foregoing, it should also be understood that any of the various buttress assemblies described herein may be further constructed and operable in accordance with at least some of the teachings of U.S. Pat. Pub. No. 2016/0278774, entitled "Method of Applying a Buttress to a Surgical Stapler," published September 29, 2016, the disclosure of which is incorporated by reference herein; U.S. Pat. Pub. No. 2017/0049444, entitled "Implantable Layers for a Surgical Instrument," published February 23, 2017, the disclosure of which is incorporated by reference herein; U.S. Pat. Pub. No. 2017/0086837, entitled "Compressible Adjunct with Crossing Spacer Fibers," published March 30, 2017, the disclosure of which is incorporated by reference herein; and U.S. Pat. Pub. No. 2017/0086842, entitled "Method for Applying an Implantable Layer to a Fastener Cartridge," published March 30, 2017, the disclosure of which is incorporated by reference herein. Furthermore, in addition to the methods described herein, any of the various buttress assemblies described herein may be applied to end effector (40) in accordance with at least some of the teachings of U.S. Pat. Pub. No. 2017/0056016, entitled "Surgical Stapler Buttress Applicator with End Effector Actuated Release Mechanism," published March 2, 2017, the disclosure of which is incorporated by reference herein; U.S. Pat. Pub. No. 2017/0056017, entitled "Surgical Stapler Buttress Applicator with Multi-Zone Platform for Pressure Focused Release," published March 2, 2017, the disclosure of which is incorporated by reference herein; U.S. Pat. Pub. No. 2017/0055980, entitled "Surgical Stapler Buttress Applicator with Spent Staple Cartridge Lockout," published March 2, 2017, the disclosure of which is incorporated by reference herein; U.S. Pat. Pub. No. 2017/0056018, entitled "Surgical Stapler Buttress Applicator with State Indicator," published March 2, 2017, the disclosure of which is incorporated by reference herein; U.S. Pat. Pub. No. 2017/0055982, entitled "Surgical Stapler Buttress Applicator with Multi-Point Actuated Release Mechanism," published March 2, 2017, the disclosure of which is incorporated by reference herein; U.S. Pat. Pub. No. 2017/0055981, entitled "Method of Applying a Buttress to a Surgical Stapler End Effector," published March 2, 2017, the disclosure of which is incorporated by reference herein; and/or U.S. Pat. Pub. No. 2017/0086842, entitled "Method for Applying an Implantable Layer to a Fastener Cartridge," published March 30, 2017, the disclosure of which is incorporated by reference herein. Various suitable ways in which the teachings herein may be combined with various teachings of the above-cited references will be apparent to those of ordinary skill in the art.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI™ system by Intuitive Surgical, Inc., of Sunnyvale, California.

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

### The below are numbered clauses of the invention:

1. A buttress assembly for reinforcing tissue layers joined by surgical stapling, wherein the buttress assembly comprises:
   (a)a multi-layer base material comprising:
      (i) a mesh layer comprising a first mesh surface and a second mesh surface, wherein the first mesh surface is configured to contact the tissue joined by the surgical stapling,
      (ii) a first film layer contacting the second mesh surface of the mesh layer, wherein the first film layer is configured as an adhesive layer, and
      (iii) a second film layer comprising a first film surface and a second film surface, wherein first film surface contacts the first film layer, wherein the second film surface is configured to face away from the tissue joined by the surgical stapling; and
   (b)an adhesive located on the second film surface of the second film layer, wherein the adhesive is configured to provide releasable adhesion of the buttress assembly with an end effector of a surgical stapler.
2. The buttress assembly of clause 1, wherein the mesh layer comprises a knitted textile constructed from an absorbable synthetic material.
3. The buttress assembly of clause 2, wherein the mesh layer comprises polyglactin 910.
4. The buttress assembly of clause 2, wherein the mesh layer comprises a multi-filament structure.
5. The buttress assembly of clause 1, wherein the first film layer comprises pol ydioxanone.
6. The buttress assembly of clause 5, wherein the first film layer has a thickness of about eight micrometers.
7. The buttress assembly of clause 1, wherein the second film layer comprises an absorbable synthetic material.
8. The buttress assembly of clause 7, wherein the second film layer comprises poliglecaprone 25.
9. The buttress assembly of clause 8, wherein the second film layer has a thickness of about ten micrometers.
10. The buttress assembly of clause 1, wherein the second film layer is configured as an adhesion barrier to prevent tissue adhesions.
11. The buttress assembly of clause 1, wherein the multi-layer base material is formed by laminating the mesh layer, the first film layer, and the second film layer together by applying a predetermined pressure and predetermined heat over a predetermined time.
12. The buttress assembly of clause 1, wherein the multi-layer base material of the buttress assembly comprises cut edges.
13. The buttress assembly of clause 12, wherein the mesh layer defines a knit pattern, wherein the knit pattern is oriented at about 45 degrees relative to a longitudinal axis of the buttress assembly.
14. The buttress assembly of clause 1, wherein the mesh layer comprises a plurality of openings that are configured to promote tissue ingrowth.
15. The buttress assembly of clause 14, wherein the plurality of openings is defined by a knitted textile constructed from an absorbable synthetic material.
16. A buttress configured for use with an end effector of a surgical stapler, wherein the buttress is further configured for reinforcing a surgically stapled site, the buttress comprising:
   (a)a mesh layer knitted from a multi-filament absorbable synthetic material, wherein the mesh layer defines a plurality of openings, wherein on a first side of the mesh layer the plurality of openings is configured to face the surgically stapled site and contact tissue to promote tissue ingrowth; and
   (b)a film layer located opposite to the first side of the mesh layer with the exposed plurality of openings, wherein the film layer is configured to face away from the surgically stapled site and prevent tissue adhesions at and around the surgically stapled site.
17. The buttress of clause 16, wherein the mesh layer comprises polyglactin 910, and wherein the film layer comprises poliglecaprone 25.
18. The buttress of clause 17, further comprising an adhesive layer positioned between the mesh layer and the film layer, wherein the adhesive layer is configured to adhesively bond the mesh layer with the film layer to form a laminate, wherein the adhesive layer comprises polydioxanone.
19. A method of reinforcing tissue at a surgically stapled site, the method comprising:
   (a)positioning an end effector of a surgical stapler relative to a buttress applicator, wherein the buttress applicator selectively retains a buttress comprising:
      (i) a mesh layer knitted from a multi-filament absorbable synthetic material, wherein the mesh layer defines a plurality of openings, wherein on a first side of the mesh layer the plurality of openings is configured to face the surgically stapled site and contact tissue to promote tissue ingrowth, and
      (ii) a film layer located opposite to the first side of the mesh layer with the exposed plurality of openings, wherein the film layer is configured to face away from the surgically stapled site and prevent tissue adhesions at and around the surgically stapled site;
   (b)clamping the end effector onto a portion of the buttress applicator, wherein the act of clamping causing disengagement of retention features of the buttress applicator from the buttress, and wherein the act of clamping further causes the buttress to releasably attach with the end effector via an adhesive located on the buttress;
   (c) separating the end effector from the buttress applicator;
   (d)positioning the end effector relative to the tissue to be surgically stapled; and
   (e)stapling the tissue, wherein the act of stapling the tissue comprises applying the buttress to the surgically stapled site with the first side of the mesh layer facing the surgically stapled site, and with the film layer facing away from the surgically stapled site to act as an adhesion barrier.
20. The method of clause 19, further comprising prior to the buttress being loaded onto the buttress applicator, the buttress is mechanical cut from a larger piece, wherein the cutting avoids overheating the buttress, thereby preventing charred edges.

## Claims

1. A buttress assembly for reinforcing tissue layers joined by surgical stapling, wherein the buttress assembly comprises:
(a)a multi-layer base material comprising:
(i) a mesh layer comprising a first mesh surface and a second mesh surface, wherein the first mesh surface is configured to contact the tissue joined by the surgical stapling,
(ii) a first film layer contacting the second mesh surface of the mesh layer, wherein the first film layer is configured as an adhesive layer, and
(iii) a second film layer comprising a first film surface and a second film surface, wherein first film surface contacts the first film layer, wherein the second film surface is configured to face away from the tissue joined by the surgical stapling; and
(b)an adhesive located on the second film surface of the second film layer, wherein the adhesive is configured to provide releasable adhesion of the buttress assembly with an end effector of a surgical stapler.

2. The buttress assembly of claim 1, wherein the mesh layer comprises a knitted textile constructed from an absorbable synthetic material.

3. The buttress assembly of claim 2, wherein the mesh layer comprises polyglactin 910, and/or wherein the mesh layer comprises a multi-filament structure.

4. The buttress assembly of any of claims 1 to 3, wherein the first film layer comprises polydioxanone.

5. The buttress assembly of any of claims 1 to 4, wherein the first film layer has a thickness of about eight micrometers.

6. The buttress assembly of any of claims 1 to 5, wherein the second film layer comprises an absorbable synthetic material, preferably wherein the second film layer comprises poliglecaprone 25.

7. The buttress assembly of any of claims 1 to 6, wherein the second film layer has a thickness of about ten micrometers.

8. The buttress assembly of any of claims 1 to 7, wherein the second film layer is configured as an adhesion barrier to prevent tissue adhesions.

9. The buttress assembly of any of claims 1 to 8, wherein the multi-layer base material is formed by laminating the mesh layer, the first film layer, and the second film layer together by applying a predetermined pressure and predetermined heat over a predetermined time.

10. The buttress assembly of any of claims 1 to 9, wherein the multi-layer base material of the buttress assembly comprises cut edges.

11. The buttress assembly of any of claims 1 to 10, wherein the mesh layer defines a knit pattern, wherein the knit pattern is oriented at about 45 degrees relative to a longitudinal axis of the buttress assembly.

12. The buttress assembly of any of claims 1 to 11, wherein the mesh layer comprises a plurality of openings that are configured to promote tissue ingrowth, preferably wherein the plurality of openings is defined by a knitted textile constructed from an absorbable synthetic material.

13. A buttress configured for use with an end effector of a surgical stapler, wherein the buttress is further configured for reinforcing a surgically stapled site, the buttress comprising:
(a) a mesh layer knitted from a multi-filament absorbable synthetic material, wherein the mesh layer defines a plurality of openings, wherein on a first side of the mesh layer the plurality of openings is configured to face the surgically stapled site and contact tissue to promote tissue ingrowth; and
(b) a film layer located opposite to the first side of the mesh layer with the exposed plurality of openings, wherein the film layer is configured to face away from the surgically stapled site and prevent tissue adhesions at and around the surgically stapled site.

14. The buttress of claim 13, wherein the mesh layer comprises polyglactin 910, and wherein the film layer comprises poliglecaprone 25, preferably wherein the buttress further comprises an adhesive layer positioned between the mesh layer and the film layer, wherein the adhesive layer is configured to adhesively bond the mesh layer with the film layer to form a laminate, wherein the adhesive layer comprises polydioxanone.
